# EUROPÄISCHE PATENTSCHRIFT

(11) **EP 3 087 371 B1**
(45) Veröffentlichungstag und Bekanntmachung des Hinweises auf die Patenterteilung: **29.04.2020**
(21) Anmeldenummer: 14838912.5
(22) Anmeldetag: 23.12.2014
(51) Int. Cl.: G01N 21/3586, G01N 21/3563, G01N 33/00, B07C 5/342

(54) **VERFAHREN ZUR KLASSIFIZIERUNG VON SAATGUT**
METHOD FOR CLASSIFYING SEEDS
PROCEDE POUR LA CLASSIFICATION DE SEMENCES

(30) Priorität: 24.12.2013 DE 102013021898
(43) Veröffentlichungstag der Anmeldung: 02.11.2016
(73) Patentinhaber: KWS SAAT SE & Co. KGaA, 37574 Einbeck (DE)
(72) Erfinder: HILSCHER, Elke, 37574 Einbeck (DE); FRIEDHOFF, Frank, 37574 Einbeck (DE); HIRSCHMANN, Christian, 37574 Einbeck (DE)
(86) Internationale Anmeldenummer: PCT/DE2014/000644
(87) Internationale Veröffentlichungsnummer: WO 2015/096827

(56) Entgegenhaltungen:
- US-A1- 2004 267 457
- US-A1- 2013 126 399
- GUA TIANTIAN ET AL: "Evaluation of wheat seeds by terahertz imaging", 2013 6TH UK, EUROPE, CHINA MILLIMETER WAVES AND THZ TECHNOLOGY WORKSHOP (UCMMT), IEEE, 9. September 2013 (2013-09-09), Seiten 1-2, XP032510392, DOI: 10.1109/UCMMT.2013.6641513 [gefunden am 2013-10-18]
- JINHAI SUN ET AL: "Identifying type of maize with terahertz time-domain spectroscopy", BIOMEDICAL ENGINEERING AND INFORMATICS (BMEI), 2010 3RD INTERNATIONAL CONFERENCE ON, IEEE, PISCATAWAY, NJ, USA, 16. Oktober 2010 (2010-10-16), Seiten 918-921, XP031804429, ISBN: 978-1-4244-6495-1
- PALKA, N. ET AL: "Terahertz Spectra of explosives measured by optical parametric oscillator-based system and time domain spectroscopy", ACTA PHYSICA POLONICA, Bd. 122, Nr. 5, 2012, Seiten 946-949, XP002738075, poland

## Beschreibung

Die vorliegende Erfindung betrifft Verfahren zur Klassifizierung und zur Sortierung von Saatgut. Insbesondere betrifft die Erfindung Verfahren zur Klassifizierung und/oder zur Sortierung von Saatgut unter Verwendung von Strahlung im Terahertz-Bereich, beispielsweise mittels der Terahertz-Zeitbereich-Spektroskopie.

In der Landwirtschaft besteht seitens der Landwirte ein Bedarf an qualitativ hochwertigem Saatgut. Qualitativ hochwertiges Saatgut zeichnet sich unter anderem durch hohe Keimfähigkeit, hohe Triebkraft und homogenes Auflaufverhalten aus. Um qualitativ hochwertiges Saatgut liefern zu können, müssen Saatguthersteller ihr Saatgut klassifizieren und sortieren, um qualitativ hochwertiges von weniger hochwertigem Saatgut unterscheiden und trennen zu können. Üblicherweise werden für die Klassifizierung und/oder Sortierung von Saatgut einfach bestimmbare physikalische Unterschiede im Phänotyp genutzt, beispielsweise Größenunterschiede und/oder Gewichtsunterschiede zwischen Saatgut-Körnern, die einen Embryo enthalten und Saatgut-Körnern, die keinen Embryo enthalten.

Um qualitativ möglichst hochwertiges Saatgut bereitzustellen, ist jedoch eine feinere Differenzierung des Saatgutes erforderlich. Beispielsweise kann das Saatgut von Zuckerrüben *(Beta vulgaris)* in sechs verschiedene Klassen eingeteilt werden:
Klasse 1 - leere Körner (ohne Embryo)
Klasse 2 - volle Körner (mit einem vollständig ausgebildeten Embryo)
Klasse 3 - leicht geschrumpft (mit einem leicht geschrumpften Embryo)
Klasse 4 - stark geschrumpft (mit einem stark geschrumpften Embryo)
Klasse 5 - Zwilling (mit 2 Embryonen in einer Samenschale)
Klasse 6 - Bigerme (mit 2 Embryos in zwei Samenschalen)

Bei der Sortierung von Saatgut ist es Ziel, möglichst nur Körner mit einem vollständig ausgebildeten Embryo in der "Gutware" zu erhalten. In Bezug auf das vorgenannte Beispiel des Zuckerrüben-Saatguts bedeutet das, dass sowohl Körner der Klasse 1 wie auch Körner der Klassen 3 bis 6 aussortiert werden sollten. Ferner ist sicherzustellen, dass bei der Sortierung nicht zu große Mengen an Körner der Klasse 2 fälschlicherweise als "Schlechtware" aussortiert werden.

Da Saatgut-Körner in ihrer Beschaffenheit sehr unterschiedlich sind, wird das vorgenannte Ziel nur dann erreicht, wenn die Selektionsgrenzen für das Sortieren des Saatguts in die einzelnen Klassen sehr eng und mit wenig Toleranz eingestellt werden. Zur Einstellung der Selektionsgrenzen sowie zur Überprüfung der Aufbereitungsqualität werden regelmäßig Proben gezogen und einer Röntgen-Diagnostik mit manueller Bildauswertung zugeführt. Eine Automatisierung oder personalunabhängige Auswertung dieser Qualitätskontrolle ist bislang nicht möglich. Zudem erfordert die gesundheitsschädliche ionisierende Strahlung bei der Qualitätskontrolle mittels Röntgen-Diagnostik umfangreiche Schutzmaßnahmen für das Personal. Außerdem ist eine schädliche Auswirkung der ionisierenden Röntgenstrahlung auf das Saatgut ebenfalls nicht auszuschließen. Daher wird die Röntgen-Diagnostik auch nicht bei der Aufbereitung von Saatgut und dessen Sortierung selbst eingesetzt, sondern nur zur Prüfung der Qualität der Beprobung des entsprechend den Klassen sortierten Saatguts.

Als Alternative zur Qualitätskontrolle von Saatgut mittels Röntgen-Diagnostik ist die Computer-Tomographie bekannt (Maisl, M. et al., "Automatisches CT-System mit integrierter Auswertung", DACH-Jahrestagung 2012 in Graz - Mi.1.B.1, Seiten 1-7). Allerdings ist das dort vorgeschlagenen CT-System mit automatischem Probenwechsler und automatischer Bildverarbeitung sehr aufwändig und auch nur begrenzt automatisierbar, so dass auch dieses Verfahren nur bei der Prozesskontrolle zur Überprüfung der Aufbereitungsqualität im Rahmen der Produktion von Zuckerrübensamen zum Einsatz kommt, nicht aber bei der Saatgut-Aufbereitung selbst.

Andere Verfahren wie die Pulsthermographie, ein bekanntes zerstörungsfreies Prüfverfahren, das in vielen Bereichen den herkömmlichen Techniken, wie Röntgen, Ultraschall und Wirbelstrom in der Aussagekraft überlegen ist, konnte sich jedoch bei der Sortierung und Klassifizierung von Saatgut nicht durchsetzen.

Weiterhin offenbart US 2013/0229647 A1 ein Verfahren zur Klassifizierung von Samen, insbesondere von Zuckerrübensamen, welches anhand von Informationen aus aufgezeichneten Spektren im IR-Frequenzbereich eine Klassierung des Samens auf Grundlage von Unterschieden in der Ausbildung des im Samen enthaltenen Embryos zulassen. Jedoch ist die Aussagekraft der IR-Spektren nur begrenzt, sodass häufig eine zuverlässig Zuordnung eines Samens in eine definierte Klasse nicht möglich ist. Das Dokument "Evaluation of wheat seeds by terahertz imaging", Tiantian Guo et al., in "Millimeter Waves and THz Technology Workshop, 9-11 Sept. 2013, Rome, Italy offenbart eine Verwendung von THz Spektoskopie zur Klassifizierung von Getreide.

Die Aufgabe bestand daher darin, ein Verfahren bereitzustellen, das eine automatische Klassifizierung und/oder Sortierung von Saatgut anhand der verschiedenen, die Klassen des jeweiligen Saatgutes definierenden Parameter ermöglicht, und das vorzugsweise nicht nur in der Prozesskontrolle, sondern auch in der Saatgut-Aufbereitung selbst einsetzbar ist, um dort einen höhere Sortiergenauigkeit bei höherer Ausbeute zu erzielen.

Die Aufgabe wird durch ein Verfahren nach Anspruch 1 zur Klassifizierung und/oder zur Sortierung von Saatgut unter Verwendung von Strahlung im Terahertz-Bereich, mittels der Terahertz-Zeitbereich-Spektroskopie gelöst.

Die Terahertz-Zeitbereich Spektroskopie (terahertz time-domain spectroscopy, THz-TDS) nutzt Terahertz-Strahlung zur Untersuchung von Proben. Diese Technik ist geeignet Parameter verschiedenster Materialen quantitativ und qualitätiv zu bestimmen (Duvillaret, L., Garet, F., & Coutaz, J. L. (1996). A reliable method for extraction of material parameters in terahertz time-domain spectroscopy. Selected Topics in Quantum Electronics, IEEE Journal of, 2(3), 739-746; Dorney, T. D., Baraniuk, R. G., & Mittleman, D. M. (2001). Material parameter estimation with terahertz time-domain spectroscopy. JOSA A, 18(7), 1562-1571). Dabei ist Terahertz-Strahlung elektromagnetische Strahlung, die im elektromagnetischen Spektrum zwischen der Infrarotstrahlung und der Mikrowellenstrahlung liegt. Terahertz-Strahlung besteht aus elektromagnetischen Wellen mit Frequenzen zwischen 100 Gigahertz (GHz) und 10 Terahertz (THz).

Bei der Terahertz-Zeitbereich-Spektroskopie wird eine zu untersuchende Probe einem sehr kurzen Puls elektromagnetischer Strahlung mit einer Frequenz im Terahertz-Bereich ausgesetzt. Dazu wird der sehr kurze Puls Terahertz-Strahlung mit einem sehr kurz gepulsten Laser erzeugt, denn ultrakurze Laserpulse mit einer Dauer von einigen Femtosekunden (1 fs = 10⁻¹⁵ s) können in Halbleitern oder nichtlinear optischen Materialien Terahertzpulse im Pikosekundenbereich (1 ps = 10⁻¹² s) erzeugen, die nur aus ein bis zwei Zyklen der elektromagnetischen Schwingung bestehen. Durch elektrooptische Methoden können sie auch kohärent, das heißt zeitaufgelöst, gemessen werden.

Bei der Terahertz-Zeitbereich-Spektroskopie wird ein Puls, der THz-Puls, in zwei Teile aufgeteilt, von denen einer zur Erzeugung und der andere zur Detektion verwendet wird. Durch die relative zeitliche Verzögerung kann der THz-Puls abgetastet werden. Mittels kohärentem Nachweis wird das elektrische Feld des THz-Pulses detektiert, also die THz-Welle in Amplitude und Phase erfasst. Mit Hilfe einer Fourier-Analyse des aufgenommenen THz-Signals lassen sich spektral breitbandige Aussagen über die zu untersuchende Probe machen, welche dann Rückschlüsse auf Material-Parameter zulassen.

Üblicherweise wird das Signal des erzeugte THz-Puls, der Frequenzkomponenten enthalten kann, mit denen der gesamte Terahertz-Bereich abgedeckt werden kann, nach Reflexion an einer Probe oder nach Transmission durch die Probe gemessen und mit dem Eingangspuls als Referenz verglichen. Für eine Auswertung des Signals können die Signal-Amplitude, die zeitliche Verzögerung, die Phase und/oder das Spektrum genutzt werden. Die Änderung der Amplitude gibt beispielsweise Auskunft über die Probenbeschaffenheit wie Porosität, Absorption, Dicke und Homogenität. Die Verzögerung des THz-Pulses beim Durchgang durch die Probe kann durch die optische Dicke der Probe (Brechungsindex n mal geometrischer Dicke d) bedingt sein. Durch Mehrfachreflexionen innerhalb der Probe können zusätzliche Echopulse auftreten, die abhängig von der Probendicke mehr oder weniger überlagert sind. Die Feldoszillationen im Anschluss an den Hauptpuls beinhalten die spektralen Informationen der Probe, die durch eine FourierTransformation der zeitlichen Wellenform zugänglich sind.

Es ist bekannt, dass Terahertz-Strahlung verwendet werden kann, um den Wassergehalt von Pflanzen zu bestimmen, insbesondere den Wassergehalt von Blättern (Jördens et al. J. Biol. Phys. 35: 255-264 (2009); Gente et al. J. Infrared Milli Terahz Waves 34: 316-323 (2013); JP 2009/042207 A). Ferner wurde mit der Terahertz-Spektroskopie die Dichte von Holz bestimmt (Koch et al. Wood Sc. and Techn. 32: 421-427 (1998)) oder der Befall von Holz mit Nematoden ermittelt (Liang et al. PIERS Proceedings, Taipei, March 25-28, 2013: 430-433).

Die vorliegende Erfindung basiert auf der Verwendung der Terahertz-Zeitbereich-Spektroskopie zur Charakterisierung von Saatgut. Dabei betrifft die Charakterisierung Parameter, die eine Zuordnung in unterschiedliche Qualitätsklassen erlauben. Solche Parameter sind insbesondere morphologische Strukturen des Saatguts wie das Vorhandensein bestimmter struktureller Elemente im Saatkorninneren (z.B. das Vorhandensein eines Embryos) oder die Anordnung von strukturellen Elementen in Saatkorninneren (z.B. 2 Embryonen in einer oder in zwei Samenschalen).

In einem ersten Aspekt der Erfindung wird ein Verfahren zur Klassifizierung und/oder Sortierung von Saatgut bereitgestellt, bei dem Saatgut unter Verwendung von Strahlung im Terahertz-Bereich, beispielsweise mittels der Terahertz-Zeitbereich-Spektroskopie klassifiziert und/oder sortiert wird.

Das Verfahren zur Klassifizierung und/oder Sortierung von Saatgut gemäß der Erfindung kann die folgenden Schritte umfassen:
- Beaufschlagen des Saatgut-Korns mit einem Terahertz-Puls,
- Messen des Signals, das der Terahertz-Puls nach Transmission und/oder Reflexion durch das Saatgut-Korn erzeugt,
- Bestimmen der durch die Transmission und/oder Reflexion bedingten Amplitude, zeitlichen Verzögerung, Phase und/oder des Spektrums des Signals und
- Zuordnen des Saatgut-Korns zu einer vorbestimmten Saatgutklasse

Weiterhin kann das Verfahren zur Klassifizierung und/oder Sortierung von Saatgut gemäß der Erfindung auch einen vorgeschalteten Schritt des Einbringens eines Saatgut-Korns in den Messbereich eines Terahertz-Zeitbereich-Spektrometers und/oder einen nachgeschalteten Schritt des Ausführens des Saatgut-Korns aus dem Messbereich des Terahertz-Zeitbereich-Spektrometers aufweisen.

In einer Ausführungsform des Verfahrens erfolgt das Zuordnen des Saatgut-Korns zu einer vorbestimmten Saatgut-Klasse auf Basis einer Kalibrierung der Terahertz-Zeitbereich-Spektroskopie anhand von Referenzwerten und/oder Referenzkörnern für zumindest eine der Saatgut-Klassen, vorzugsweise für die gewünschte Saatgut-Klasse, also die Saatgut-Klasse mit dem qualitativ hochwertigsten Saatgut. In einer anderen Ausführungsform erfolgt die Kalibrierung der Terahertz-Zeitbereich-Spektroskopie anhand von Referenzwerten und/oder Referenzkörnern für jede der vorgesehenen Saatgut-Klassen.

Durch die Klassifizierung mehrerer Saatgut-Körner einer Probe kann die Qualität der Probe bestimmt werden, indem festgestellt wird, wie hoch der Anteil an Saatgut-Körner jeder Saatgut-Klasse in der Probe ist. Dabei erfolgt die Klassifizierung der einzelnen Saatgut-Körner aus der Mehrzahl von Saatgut-Körner in einem Terahertz-Zeitbereich-Spektrometer nacheinander.

In einer zusätzlichen und/oder alternativen Ausführungsform wird das Saatgut-Korn nach dem Ausführen aus dem Messbereich des Terahertz-Spektrometers entsprechend seiner Klassifizierung einsortiert. Diese Ausführungsform ermöglicht das Aussortieren der Saatgut-Körner, die nicht in die gewünschte Saatgut-Klasse gehören. Somit lässt sich mit dem Verfahren auch die Qualität des Saatguts verbessern, weil nur die Saatgut-Körner der gewünschten Klasse für die weitere Saatgut-Aufbereitung oder -produktion verwendet werden können. Saatgut-Körner, die nicht in die gewünschte Saatgut-Klasse gehören, können vor der weiteren Saatgut-Aufbereitung aussortiert werden.

Das Verfahren zur Klassifizierung und/oder Sortierung von Saatgut gemäß der Erfindung weist eine Sortiergenauigkeit vorzugsweise von mindestens 75%, 76%, 77%, 78%, 79% oder 80%, besonders bevorzugt von mindestens 81%, 82%, 83%, 84%, 85%, 86%, 87%, 88%, 89% oder 90%, und ganz besonders bevorzugt von mindestens 90%, 91%, 92%, 93%, 94%, 95%, 96%, 97%, 97,5%, 98%, 98,5%, 99%, 99,5% oder 100% auf.

In einer zusätzlichen und/oder alternativen Ausführungsform des Verfahrens wird das mit Hilfe der Terahertz-Zeitbereich-Spektroskopie zu klassifizierende und/oder sortierende Saatgut vorsortiert, beispielsweise nach der Größe der Saatgut-Körner. Auf diese Weise können dem Verfahren zur Klassifizierung und/oder Sortierung von Saatgut nur Saatgut-Körner des gleichen oder annähernd gleichen Kalibers zugeführt werden. In dieser Ausführungsform wird das Saatgut vor seiner Klassifizierung also kalibriert. Diese Ausführungsform kann bei Bedarf die Genauigkeit der Saatgut-Klassifizierung und/oder -Sortierung noch weiter erhöhen.

Bei einer Ausführungsform des Verfahrens gemäß dem ersten Aspekt der Erfindung weist die THz-Strahlung des THz-Pulses eine Frequenz von mindestens 0,1 THz, vorzugsweise von mindestens 0,5 THz, besonders bevorzugt von mindestens 1 THz und ganz besonders bevorzugt von mindestens 5 THz auf.

In einer alternativen und/oder zusätzlichen Ausführungsform weist die THz-Strahlung des THz-Pulses eine Frequenz von nicht mehr als 10 THz, vorzugweise von nicht mehr als 6 THz, besonders bevorzugt von nicht mehr als 4 THz und ganz besonders bevorzugt von nicht mehr als 3 THz auf.

In einer zusätzlichen und/oder alternativen Ausführungsform weist der Terahertz-Puls eine Dauer im Bereich von etwa 3ps bis etwa 25 ps auf, vorzugsweise von etwa 5 ps bis etwa 20 ps, und bevorzugt von etwa 15 ps auf. Besonders bevorzugt handelt es sich um einen Femtosekundenlaser, welcher linear polarisiert ist, welcher bei 1560 nm emittiert oder welcher mit einer Wiederholfrequenz von 100 MHz bei einer durchschnittlichen Laserausgabeleistung von >60 mW arbeitet. Ferner besonders bevorzugt ist eine Pulslänge nach 2,5 m Faseroptik von <90 fs.

In einer zusätzlichen und/oder alternativen Ausführungsform weist das Terahertz-Spektrometer eine oder mehrere der folgenden Komponenten auf: eine Laserquelle, vorzugsweise mit Faseroptik, eine Verzögerungsstrecke mit einem Abtastbereich von vorzugsweise 0-300 ps, eine Terahertz-Emitterantenne und Terahertz-Empfängerantenne, eine terahertzfähige Optik, ein Lock-in-Verstärker und/oder einem Recheneinheit (Computer) mit Messprogramm zur Datenerfassung und -auswertung. Besonders bevorzugt bietet das Spektrometer einen dynamischen Bereich von >70 dB und einen Spektralbereich von >3,5 THz.

In einer besonders bevorzugten Ausführungsform erfolgt eine Terahertz-Messung mit folgenden Einstellungen: Ein spektraler Messbereich von 0,01-10 THz und eine Verzögerung von 0-104 ps mit einem Intervall von 0,05 ps. Die Integrationszeit pro Verzögerungsstellung beträgt 30 msec, womit sich eine Gesamtmesszeit pro Saatgutkorn von ca. 1 Minute ergibt.

Da die Terahertz-Zeitbereich-Spektroskopie eine berührungslose, zerstörungsfreie Methode ist, kann prinzipiell jedes Saatgut mit dessen Hilfe klassifiziert und/oder sortiert werden.

Bei einer Ausführungsform des Verfahrens nach dem ersten Aspekt der Erfindung ist das Saatgut aus der Gruppe von Früchten ausgewählt, bei denen das Perikarp oder ein Teil des Perikarps, also das Endokarp, das Mesokarp und/oder das Exokarp verholzt ist.

In einer zusätzlichen und/oder alternativen Ausführungsform ist das Saatgut aus der Gruppe ausgewählt, die Gemüsesaat, Getreidesaat, Körner von Steinfrüchten, Körner von Beeren, Nüsse und Körner von Fuchsschwanzgewächsen umfasst.

In einer zusätzlichen und/oder alternativen Ausführungsform ist die Gemüsesaat aus der Gruppe ausgewählt, die Saatgut von Salaten, des von Kohl, von Blütengemüse, von Fruchtgemüse, von Knollengemüse, von Zwiebelgemüse, der Hülsenfrüchte und anderen Gemüsen umfasst.

Die Salate, umfassen
- Gartensalat (*Lactuca sativa* L*.*), *beispielsweise.* Kopfsalat (*Lactuca sativa* L. var. *capitata* L.), Schnittsalat (*Lactuca sativa* L. var. *crispa* L.). Bindesalat (*Lactuca sativa* L. var. *longifolia* L.), Spargelsalat (*Lactuca sativa* var. *angustana*) und Eisbergsalat,
- Gemeine Wegwarte (*Cichorium intybus L*.), *beispielsweise* Chicoree (*Cichorium intybus* var. *foliosum*) und Endivie (*Cichorium endivia L*.),
- Rucola, Rauke (*Diplotaxis tenuifolia* oder *Eruca sativa*), Mangold (*Beta vulgaris* subsp. *vulgaris*), Spinat (*Spinacia oleracea L*.), Wasserspinat (*Ipomoea aquatica FORSSK*.), Gartenmelde (*Atriplex hortensis L*.), Brunnenkresse (*Nasturtium officinale R.BR*.), Portulak (*Portulaca ssp. sative (HAW.) ČEL*.), Gewöhnliches Tellerkraut (=Kuba-Spinat, Winterportulak, *Claytonia perfoliata DONN. EX WILLD*), Malabar-Spinat, Indischer Spinat (*Basella alba*)*,* Neuseeländer Spinat, Neuseelandspinat (*Tetragonia tetragonioides*), Jambú (*Acmella oleracea (L.) R.K.JANSEN*), Jute-Blätter (*Corchorus olitorius L*.), Eiskraut (*Mesembryanthemum crystallinum*), Taglilien, z. B. die Gelbrote Taglilie (*Hemerocallis fulva L*.), Ähriger Erdbeerspinat (*Chenopodium capitatum (L.) ASCH*.), Guter Heinrich (*Chenopodium bonus-henricus L*.), und Garten-Ampfer (*Rumex*).

Kohl bezeichnet Gemüse der Art Brassica, einschließlich Mittelmeer-Kohl (*B. fruticulosa*), Ruten-Kohl, oder Indischer Senf oder Serepta-Senf genannt, (*B. juncea*), Raps und Steckrübe (*B. napus*), zum Beispiel Steckrübe, Kohlrübe, Wruke (*B. napus* subsp. *rapifera MTZG*.), Raps (*B. napus* subsp. *napus L*.) und Schnittkohl (*B. napus* subsp. *pabularia*), Schwarzer Senf (*B. nigra (L.) KOCH*), Gemüsekohl (*B. oleracea* L.), zum Beispiel Blumenkohl (*B. oleracea* var. *botrytis* L.) wie Romanesco (*B. oleracea* convar. *botrytis* var. *botrytis* L.), Broccoli (*B. oleracea* var. *italica* Plenck), Kohlrabi (*B. oleracea* var. *gongylodes* L.), Kopfkohl (*B. oleracea* convar. *capitata* L.), Rotkohl (*Brassica oleracea* convar. *capitata* var. *rubra* L.), Weißkohl (*Brassica oleracea* convar. *capitata* var. *alba* L.) wie Spitzkohl, und Wirsing, Savoyer Kohl (*B. oleracea* convar. *capitata* var. *sabauda* L.), Rosenkohl (*B. oleracea* var. *gemmifera* DC.); Grünkohl, "Braunkohl" (*B. oleracea* var. *sabellica* L.); Palmkohl (*Brassica oleracea* var. *palmifolia* DC.); Markstammkohl (*B. oleracea* var. *medullosa* Thell.); Rübsen (*B. rapa L*.) wie Ölrübsen (*B. rapa* subsp. *oleifera*); Chinakohl (*B. rapa* subsp. *pekinensis*); Pak Choi (*B. rapa* subsp. *chinensis*) und Mairübe, Wasserrübe, Weiße Rübe, Teltower Rübchen, Bayerische Rübe (*B. rapa* subsp. *rapa*), beispielsweise Rübstiel.

Zum Blütengemüse zählen Artischocke (*Cynara scolymus*), Zucchini (*Curcubita pepo* subsp. *pepo* convar. *giromontiina*), Blumenkohl (*Brassica oleracea var. botrytis* L.), Broccoli (*Brassica oleracea var. italica* Plenck), Romanesco (*Brassica oleracea convar. botrytis var. botrytis*), Lilien (*Lilium L*.) (Liliengewächse) und Dahlien (*Dahlia CAV*.).

Unter Fruchtgemüse sind im Sinne der Erfindung zu verstehen:
- Wassermelone (*Citrullus lanatus (THUNB.) MATSUM. & NAKA1*.),
- Gurken (*Cucumis L*.), beispielsweise Honigmelone (*Cucumis melo L*.), Kiwano (*Cucumis metuliferus E.MEY. EX NAUDIN*) und Gurke (*Cucumis sativus L*.),
- Kürbisse und Zucchini (*Cucurbita*), beispielsweise Gartenkürbis, Zucchini, Spaghettikürbis (*C*. *pepo L*.), Riesen- oder Hokkaidokürbis (*C. maxima*), Moschuskürbis (*C. moschata*) und Feigenblatt-Kürbis (*C. ficifolia*),
- Bittermelone (*Momordica L*.), Flaschenkürbisse (*Lagenaria siceraria (MOLINA) STANDL*.), Schwammkürbis (*Luffa MILL*.), *Sechium, zum Beispiel* Chayote = Christofine, (*Sechium edule (JACQ.) SW*.), Tomate (*Solanum lycopersicum L*.), Paprika (*Capsicum L*.), Amaranth (*Amaranthus L*.), Aubergine (Solanum melongena), Avocado (*Persea americana MILL*.), Okra (*Abelmoschus esculentus (L.) MOENCH*.)*,* und Brotfrucht (*Artocarpus altitis (PARKINS. EX DUROI) FOSB. CORR. ST.JOHN*).

Zum Wurzelgemüse zählen Knollen- und Zwiebelgemüse. Knollengemüse umfasst Gelbe Rübe, Mohrrübe (*Daucus carota L.* ssp. *sativus*), Rote Bete, Rote Rübe (*Beta vulgaris* subsp. *vulgaris*), Steckrübe, Kohlrübe (*Brassica napus subsp. rapifera*), Mairübe (*Brassica rapa* subsp. *rapa* var. *majalis*) und Teltower Rübchen (*Brassica rapa* subsp. *rapa* var. *pygmaea*), Meerrettich (*Armoracia rusticana* GAERTN.MEY. & SCHERB.), Radieschen (*Raphanus sativus L.* subsp. *sativus*), Daikon (*Raphanus sativus var. longipinnatus*), Schwarzer Winter-Rettich (*Raphanus sativus* subsp. *niger* var. *niger*), Wasabi (*Wasabia japonica MATSUM*.)*,* Kartoffel (*Solanum tuberosum L*.), Garten-Schwarzwurzel (*Scorzonera hispanica L*.), Pastinake (*Pastinaca sativa*)*,* Wurzelpetersilie (*Petroselinum crispum subsp. tuberosum*), Sellerie (Apium graveolens), Kerbelrübe oder Knolliger Kälberkropf (*Chaerophyllum bulbosum* L.), Lotoswurzel (*Nelumbo*) und Yams (*Dioscorea.* L.').

Zwiebelgemüse umfasst die Lauchgewächse (*Allium*), beispielsweise *Allium*LauchgewächseZwiebel (*A. cepa L*.), Winterzwiebel, Frühlingszwiebel, (*A. fistulosum L*.), Knoblauch (*A. sativum L*.), Schalotte (*A. ascalonicum STRAND*.), Porree, Lauch (*A. porrum L*.), Perlzwiebel (*Allium porrum* var. *sectivum*) und Bärlauch (*Allium ursinum*).

Zu den Hülsenfrüchten zählen
- *Phaseolus*
   ∘ Limabohne (*Phaseolus lunatus* L.), Mondbohne
   ∘ Teparybohne (*Phaseolus acutifolius* A.GRAY)
   ∘ Feuerbohne (*Phaseolus coccineus* L.), Schminkbohne
   ∘ Gartenbohne, Buschbohne, Stangenbohne, (*Phaseolus vulgaris* L.)
      ▪ Sorten:
      ▪ Kidney-Bohne, Nierenbohne
      ▪ Perlbohne
      ▪ Wachtelbohne, Pintobohne
      ▪ Schwarze Bohne, Brasilien
      ▪ Weiße Bohne
- Sojabohne (*Glycine max* (L.) Merill)
- Erbse (*Pisum*)
   ∘ Schalerbse (*Pisum sativum* L. convar. *sativum*), auch Pahl-, Pal- oder Kneifelerbsen
   ∘ Markerbse (*Pisum sativum* L. convar. *medullare* Alef. emend. C.O. Lehm)
   ∘ Zuckererbse (*Pisum sativum* L. convar. *axiphium* Alef emend. C.O. Lehm), auch Kaiserschoten, Kiefelerbsen oder Kefen, (Zuckerschote)
   ∘ Riesenerbse (*Pisum granda sneida* L. convar. *sneidulo* p. *shneiderium*)
- Schneckenklee (*Medicago L*.)
   ∘ Gewöhnliche Luzerne, Luzerne (*M*. *sativa* L.)
- Kichererbse (*Cicer arietinum L*.)
- Linsen, (*Lens*), (*Lens culinaris* Medik.)
- Lupinen (*Lupinus L*.)
- Wicken (*Vicia L*.)
   ∘ Ackerbohne, Dicke Bohne, Saubohne (*Vicia faba L*.)
- Platterbsen (*Lathyrus L*.)
   ∘ Saat-Platterbse (*Lathyrus sativus L*.)
   ∘ Knollen-Platterbse (*Lathyrus tuberosus L*.)
- *Vigna*
   ∘ Mattenbohne, (*Vigna aconitifolia* (Jacq.) Maréchal)
   ∘ Adzukibohne, (*Vigna angularis* (Willd.) Ohwi & H. Ohashi)
   ∘ Urdbohne, (*Vigna mungo* (L.) Hepper)
   ∘ Mungbohne, (*Vigna radiata* (L.) R. Wilczek), "Sojasprossen"
   ∘ Bambara-Erdnuss, (*Vigna subterrane* (L.) Verdc.)
   ∘ Reisbohne, (*Vigna umbellata* (Thunb.) Ohwi & H. Ohashi)
   ∘ *Vigna vexillata* (L.) A. Rich., (kein deutscher Name)
   ∘ *Vigna unguiculata* (L.) Walp., in den drei Unterarten:
      ▪ Spargelbohne (*Vigna unguiculata subsp. sesquipedalis*)
      ▪ Augenbohne (*Vigna unguiculata subsp. unguiculata*)
      ▪ Catjang-Bohne (*Vigna unguiculata subsp. cylindrica*)
- Straucherbse, (*Cajanus cajan* (L.) Millsp.)
- *Macrotyloma*
   ∘ Erdbohne, (*Macrotyloma geocarpum* (Harms) Marechal & Baudet)
   ∘ Pferdebohne, (*Macrotyloma uniflorum* (Lam.) Verdc.)
- Goabohne, (*Psophocarpus tetragonolobus* (L.) DC.)
- Knollenbohne, (*Sphenostylis stenocarpa* (Hochst. ex A. Rich.) Harms)
- Faselbohne, Helmbohne, Lablab-Bohne, (*Lablab purpureus* (L.) Sweet)
- Guarbohne (*Cyamopsis tetragonolobus* (L.) Taub.)
- *Canavalia*
   ∘ Jackbohne, (*Canavalia ensiformis* (L.) DC.)
   ∘ Schwertbohne, (*Canavalia gladiata* (Jacq.) DC.).

Unter den anderen Gemüsen sind erfindungsgemäß zum Beispiel zu verstehen: Batis (*Batis* L.), Chinesische Wasserkastanie (*Eleocharis dulcis*), Eibisch (*Althaea officinalis* L.), Fenchel (*Foeniculum vulgare* (L.) Mill.), Gemüsespargel (*Asparagus officinalis* L.), Gemeiner Rhabarber (*Rheum rhabarbarum*), Japanischer Rhabarber (*Fallopia japonica* (Houtt.) Ronse Decr.), Koriander (*Coriandrum sativum* L.), Süßkartoffel (*Ipomoea batatas* L.), Quinoa (*Chenopodium quinoa* Willd.), Schwedische Rübe (*Brassica napus*), Wassermimose (*Neptunia oleracea* Lour.), Maniok, Mandioka, Kassava, Kassave oder in Lateinamerika Yuca (*Manihot esculenta* Crantz), Knolliger Sauerklee, Oca oder Yam (*Oxalis tuberosa*), Olluco, Melloco oder Knollenbaselle (*Ullucus tuberosus*), Mashua, auch Knollige Kapuzinerkresse (*Tropaeolum tuberosum*), Yacón (*Smallanthus sonchifolius*), Topinambur (*Helianthus tuberosus*) und Sonnenblume (*Helianthus annuus*).

In einer zusätzlichen und/oder alternativen Ausführungsform ist die Getreidesaat aus der Gruppe ausgewählt, die Saatgut von Weizen (*Triticum spec*.), Roggen (*Secale spec*.), Gerste (*Hordeum spec*.), Triticale, Hafer (*Avena spec*.), Mais (*Zea mays*), Reis (*Oryza spec*.), Triticale und Hirse (*Sorghum spec., Panicum spec., Pennisetum spec.* und weitere) umfasst. Dabei umfasst Weizen auch Einkorn (*T*. *monococcum*), Emmer (*T*. *dicoccum*), Hartweizen (*T*. *durum*), Kamut (*T*. *durum x polonicum*), Dinkel (*T*. *spelta*)und Weichweizen (*T*. *aestivum*). Ferner umfasst Hirse auch Rispenhirse (*Panicum miliaceum*), Kolbenhirse (*Setaria italica*), Sorghum (*Sorghum bicolor* und andere), Perlhirse (*Pennisetum glaucum*)*,* Fingerhirse (*Eleusine coracana*), Teff (*Eragrostis tef*) und Fonio (*Digitaria exilis*).

In die Gruppe der Nüsse gehören Nüsse im botanischen Sinne, wie Buchecker, Haselnuss, Walnuss (*Juglans regia*), Edelkastanie (Maroni), Eichel, Hanfnuss, Macadamianuss, Platanennuss, Steinnuss oder Wassernuss. Hierzu zählen auch Sammelnussfrüchte wie die Erdbeere. In diese Gruppe gehören auch Nüsse, die im botanischen Sinne jedoch keine Nuss sind. Das sind zum Beispiel Cashewnuss, Erdmanteln Erdnuss (*Arachis hypogaea*), Kokosnuss, Mandel, Muskatnuss, Paranuss, Pekannuss, Pistazie oder Sheanuss.

Bei den Steinfrüchten ist nur die innere Fruchtwand verholzt. Zu den Steinfrüchten gehören zum Beispiel Mango, Mirabelle, Nektarine, Pfirsich, Pflaume, Aprikose, Olive, Sauerkrische, Süßkirsche.
Sammelsteinfrucht = Himbeere, Brombeere.

Zu den Beeren zählen Bananen, Zitrusfrüchte, Datteln, Melonen, Kiwis Papayas, und Nachtschattengewächse wie Paprika, Tomate, Tamarillo, Kartoffelbeere, Aubergine.

Im Sinne der vorliegenden Erfindung gehören zu den Fuchsschwanzgewächsen beispielsweise die Zuckerrübe (*Beta vulgaris vulgaris*), Spinat, Futterrübe, Mangold und Quinoa.

Die Vorteile des erfindungsgemäßen Verfahrens liegen darin, dass keine gesundheitsschädliche und das Saatgut möglicherwiese beeinträchtigende ionisierende Strahlung für die zerstörungsfreie Analyse der Saatgut-Körner verwendet werden muss. Das Verfahren ermöglicht eine Klassifizierung von Saatgut mit hoher Genauigkeit. Zudem ist das Verfahren automatisierbar und schnell, so dass es nicht nur in der Prozesskontrolle, sondern auch im eigentlichen Klassifizierungs- und Sortierprozess des Saatgutes eingesetzt werden kann. Daher umfasst die vorliegende Erfindung auch Verfahren zur Klassifizierung und/oder Sortierung von Saatgut, bei dem die Klassifizierung und/oder Sortierung automatisiert erfolgt.

Mit dem zweiten Aspekt der Erfindung erstreckt sie sich auch auf die Verwendung der Terahertz-Zeitbereich-Spektroskopie zur Klassifizierung und/oder Sortierung von Saatgut, insbesondere zur Klassifizierung und/oder Sortierung des im Zusammenhang mit dem Verfahren nach dem ersten Aspekt der Erfindung angeführten Saatguts.

Ausgestaltungen und Ausführungsformen der vorliegenden Erfindung werden in exemplarischer Weise mit Bezug auf die angehängten Figuren beschrieben:
Figur 1: Zeitdomänenspektren von Zuckerrübensaatgut. Die Figur zeigt die Mittelwertspektren der jeweiligen Klassen.
Figur 2: Zeitdomänenspektren von Zuckerrübensaatgut. Ausschnitt von Figur 1 im Bereich von 12-24 ps. Die Figur zeigt die Mittelwertspektren der jeweiligen Klassen.
Figur 3: Zeitdomänenspektren von Zuckerrübensaatgut. Ausschnitt von Figur 1 im Bereich von 45-70 ps. Die Figur zeigt die Mittelwertspektren der jeweiligen Klassen.
Figur 4: Phasenspektren von Zuckerrübensaatgut. Die Figur zeigt die Mittelwertspektren der jeweiligen Klassen.
Figur 5: Phasenspektren von Zuckerrübensaatgut. Ausschnitt von Figur 4 im Bereich von 0,2-2 THz. Die Figur zeigt die Mittelwertspektren der jeweiligen Klassen.
Figur 6: Absorptionskoeffizientenspektren von Zuckerrübensaatgut. Die Figur zeigt die Mittelwertspektren der jeweiligen Klassen.
Figur 7: Absorptionskoeffizientenspektren von Zuckerrübensaatgut. Ausschnitt von Figur 6 im Bereich von 0,1-0,6 THz. Die Figur zeigt die Mittelwertspektren der jeweiligen Klassen.
Figur 8: Brechungsindexspektren von Zuckerrübensaatgut. Die Figur zeigt die Mittelwertspektren der jeweiligen Klassen.
Figur 9: Brechungsindexspektren von Zuckerrübensaatgut. Ausschnitt von Figur 8 im Bereich von 0,1-2 THz. Die Figur zeigt die Mittelwertspektren der jeweiligen Klassen.
Figur 10: Hauptkomponentenanalyse von Zuckerrübensaatgut: Score-Diagramm der Hauptkomponente 1 und 2.
Figur 11: Zeitdomänenspektren von Zwiebelsaatgut. Die Figur zeigt die Mittelwertspektren der beiden Klassen.
Figur 12: Zeitdomänenspektren von Zwiebelsaatgut. Ausschnitt von Figur 11 im Bereich von 12-24 ps. Die Figur zeigt die Mittelwertspektren der beiden Klassen.
Figur 13: Zeitdomänenspektren von Zwiebelsaatgut. Ausschnitt von Figur 11 im Bereich von 45-70 ps. Die Figur zeigt die Mittelwertspektren der beiden Klassen.
Figur 14: Zeitdomänenspektren von Paprikasaatgut. Die Figur zeigt die Mittelwertspektren der beiden Klassen.
Figur 15: Zeitdomänenspektren von Paprikasaatgut. Ausschnitt von Figur 14 im Bereich von 12-24 ps. Die Figur zeigt die Mittelwertspektren der beiden Klassen.
Figur 16: Zeitdomänenspektren von Paprikasaatgut. Ausschnitt von Figur 14 im Bereich von 45-70 ps. Die Figur zeigt die Mittelwertspektren der beiden Klassen.
Figur 17: Hauptkomponentenanalyse von Zwiebelsaatgut: Score-Diagramm der Hauptkomponente 1 und 2.
Figur 18: Hauptkomponentenanalyse von Paprikasaatgut: Score-Diagramm der Hauptkomponente 1 und 2.
Figur 19: Zeitdomänenspektren von Sonnenblumensaatgut. Die Figur zeigt die Mittelwertspektren der beiden Klassen.
Figur 20: Hauptkomponentenanalyse von Sonnenblumensaatgut: Score-Diagramm der Hauptkomponente 1 und 2.

### Saatgut Proben:

Die Güte und Qualität des erfindungsgemäßen Klassifikations-/Sortierungsverfahrens von Saatgut wird an Saatgut verschiedener Kulturarten wie der Zuckerrübe (*B. vulgaris*), Zwiebel (*A. cepa*), Paprika (*Capsicum*) und Sonnenblume (*H. annuus*) im Folgenden demonstriert. Die Bewertung der Eignung des erfindungsgemäßen Verfahrens basiert auf einer zuvor durchgeführten Röntgen-Diagnostik im Labor zur Klassifikation des Saatgutes. Mit der Röntgen-Diagnostik erzielte Klassierung wird im Folgenden als "Tatsächliche Klasse" benannt.

### Zuckerrübe:

Saatgut der Zuckerrübe wird aufgrund der Korngeometrie und Korndichte in 6 Kaliber eingeteilt. Die Saatgut-Körner jedes Kalibers werden einer Röntgen-Diagnostik unterzogen, die die einzelnen Saatgut-Körner in die oben beschriebenen Klassen 1 bis 6 klassiert. Im Folgenden wird Klasse 1 als "Leer", Klasse 2 als "Voll", Klasse 3 als "Leicht geschrumpft", Klasse 4 als "Stark geschrumpft", Klasse 5 als "Zwilling" und Klasse 6 als "Bigerm" bezeichnet.

### Zwiebel und Paprika:

Saatgut der Fruchtarten Zwiebel und Paprika wird anhand der Korngeometrie und Korndichte, je Fruchtart, in zwei Kaliber eingeteilt. Die Saatgut-Körner wurden ebenfalls einer Röntgen-Diagnostik unterzogen. Im Falle von Zwiebel und Paprika ist es sinnvoll das Saatgut in die Klassen "Voll" und "Geschrumpft" zu klassieren, wobei die Klasse "Geschrumpft" Körner der Klassen "Leer", "Leicht geschrumpft" und "Stark geschrumpft" enthalten konnte.

### Sonnenblume:

Bei der Sonnenblume erfolgte in Gegensatz zu den oben beschriebenen Fruchtarten keine Einteilung des Saatgutes in Kaliber. Die Saatgutkörner wurden ebenfalls der Röntgen-Diagnostik unterzogen. Analog der Zwiebel und Paprika, ist es bei der Sonnenblume sinnvoll das Saatgut in die Klassen "Voll" und "Geschrumpft" zu klassieren.

### Spezifikation des Terahertz-Spektrometers:

Die Messungen werden mit dem Terahertz-Zeitdomänenspektrometer "TERA K15" von Menlo Systems GmbH, Martinsried, Deutschland, durchgeführt. Das "TERA K15" besteht aus einer Laserquelle mit Faseroptik, einer Verzögerungsstrecke mit einem Abtastbereich von 0-300 ps, einer Terahertz-Emitterantenne TERA15-TX-FC und passendem Terahertz-Empfängerantenne TERA15-RX-FC, terahertzfähiger Optik, einem Lock-in-Verstärker TERA-C, und einem Rechner mit Messprogramm zur Datenerfassung und -auswertung.

Das Spektrometer bietet einen dynamischen Bereich von >70 dB und einen Spektralbereich von >3,5 THz. Der eingebaute Femtosekundenlaser ist linear polarisiert, emittiert bei 1560 nm, mit einer Wiederholfrequenz von 100 MHz bei einer durchschnittlichen Laserausgabeleistung von >60 mW. Die Pulslänge nach 2,5 m Faseroptik ist <90 fs.

Zur Messung der Saatgut-Körner aller Fruchtarten werden die folgenden Messeinstellungen und - parameter gewählt: Ein spektraler Messbereich von 0,01-10 THz und eine Verzögerung von 0-104 ps mit einem Intervall von 0,05 ps. Die Integrationszeit pro Verzögerungsstellung beträgt 30 msec, womit sich eine Gesamtmesszeit pro Saatgutkorn von ca. 1 Minute ergibt.

### Durchführung der Terahertz-Messungen:

Zur Messung wird jeweils ein, oben beschriebenes Saatgut-Korn in den Fokuspunkt des Terahertz-Strahls platziert. Anschließend wird das Saatgut-Korn mit einem Terahertz-Puls beaufschlagt. Der Terahertz-Puls wird von der Senderantenne generiert, passiert die Optik, wird fokussiert und interagiert mit dem Saatgut-Korn. Die Detektorantenne registriert das Signal, das der Terahertz-Puls nach Transmission und/oder Reflexion durch das Saatgut-Korn erzeugt. Typischerweise wird das Saatgut-Korn mit mehreren Terahertz-Pulsen, auch bei verschiedenen Verzögerungszeiten, beaufschlagt. Das Signal der Detektorantenne wird von einem Rechner samt Messprogramm ausgelesen und die Transmissions- und/oder Reflexionsbedingte Amplitude, die zeitlichen Verzögerung, die Phase und/oder des Spektrums des Signals bestimmt. In weiteren Schritten kann der Absorptionskoeffizient und der Brechungsindex des Saatgut-Korns berechnet werden.

### Auswertung und Ergebnisse

In der folgenden Ausführung wird beispielhaft anhand eines Kalibers das Verfahren zur Klassifikation mit Terahertz dargestellt.

### Zuckerrüben:

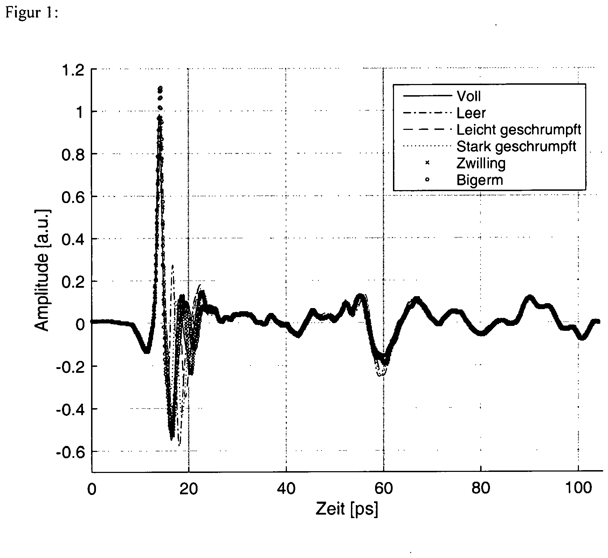

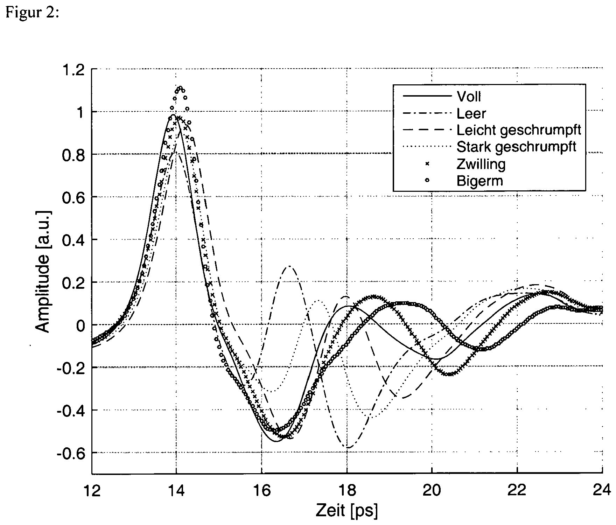

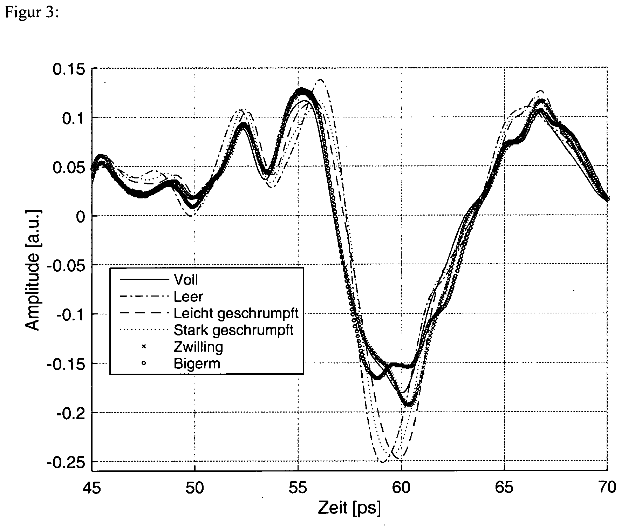

Figuren 1-3 zeigen die Zeitdomänenspektren des Saatgutes eines exemplarischen Kalibers (3,16 - 3,50 mm), wobei jeweils ein Mittelwertspektrum für jede Klasse gebildet wurde. Figur 1 zeigt das vollständige Spektrum, die Figuren 2 und 3 hingegen Ausschnitte um Unterschiede in den Spektren zu verdeutlichen. Die Spektren der sechs Klassen weisen eindeutige Unterschiede auf, beispielsweise im Bereich von 15-22 ps.

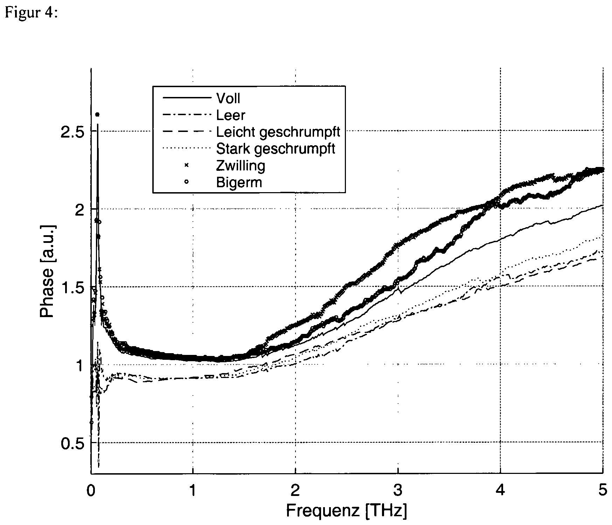

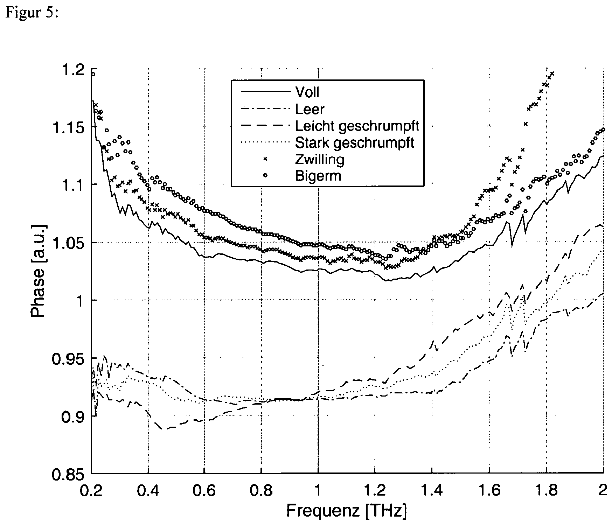

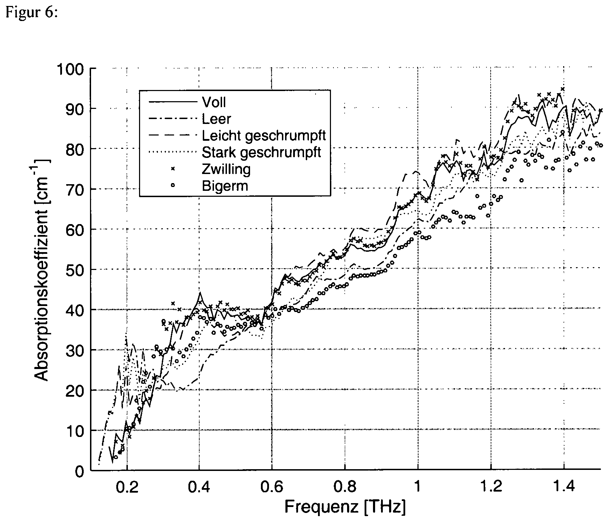

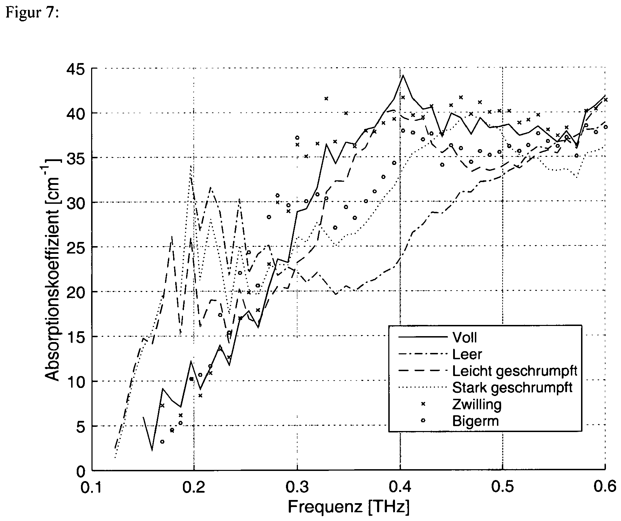

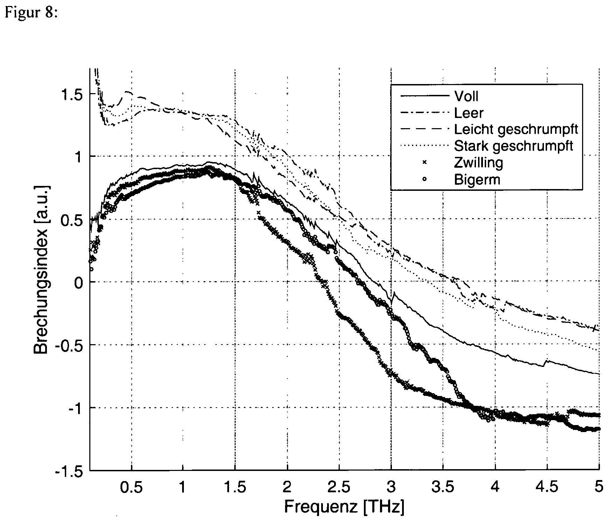

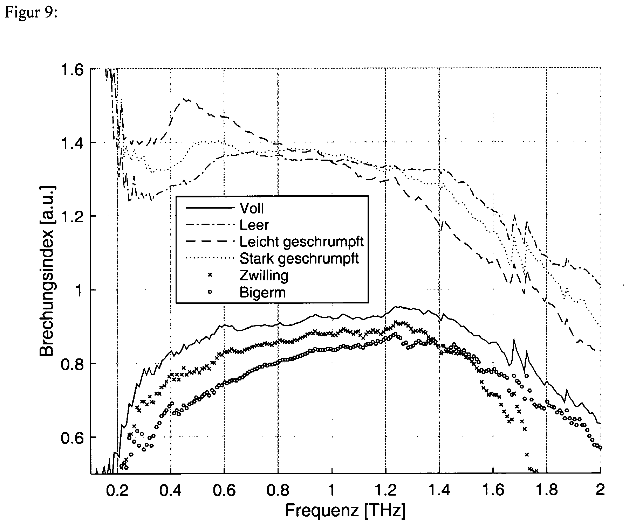

Ferner offenbaren auch die Spektren der Phase, des Absorptionskoeffizienten und des Brechungsindexes Unterschiede beim Vergleich der Klassen innerhalb eines Kalibers, wie in Figuren 4-9 dargestellt. Die Figuren 4, 6 und 8 zeigen jeweils das komplette Spektrum und die Figuren 5, 7 und 9 jeweils einen vergrößerten Ausschnitt der Phase, des Absorptionskoeffizienten und des Brechungsindexes.

Die Informationen der Zeitdomäne, der Phase, des Absorptionskoeffizienten und des Brechungsindexes und/oder eine Kombinationen davon können zur Klassifikation herangezogen werden. In der hier vorliegenden Ausführung wird beispielsweise ausschließlich das Zeitdomänenspektrum zur Klassifikation herangezogen; ebenso bei den weiteren Fruchtarten weiter unten.

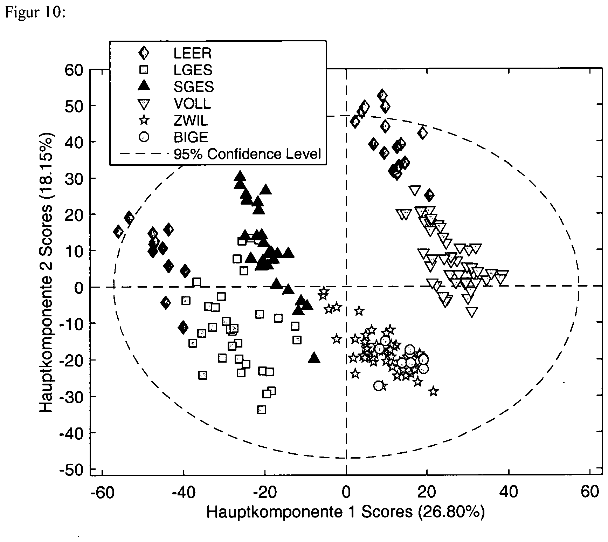

Um die Unterschiede in den Zeitdomänenspektren zu verdeutlichen und die Daten weiter zu erkunden, wird eine Hauptkomponentenanalyse (PLS Toolbox Version 7.9.1, Eigenvector Research, Inc. Wenatchee, Vereinigte Staaten von Amerika basierend auf Matlab® 2014b, The MathWorks GmbH, Ismaning, Deutschland) berechnet. Das Score-Diagramm der Hauptkomponente 1 und 2 ist in Figur 10 dargestellt. Die Klassen "Leer", "Leicht geschrumpft", "Stark geschrumpft", "Zwilling" und "Bigerm" belegen den Hauptkomponentenraum in den Quadraten II, III und IV und sind somit deutlich abgetrennt von den Klassen "Voll" und einer weiteren Gruppe der Klasse "Leer" im Quadrant I. Die Grenze zwischen den Klassen "Voll" und "Leer" im Quadrant I ist nicht scharf, d.h. einige Proben dieser beiden Klassen können überlappen.

Um die Unterschiede in den Daten zu betonen, werden die Rohdaten beispielsweise mittels Savitzky-Golay- (Savitzky,A.; Golay, M. J.E. (1964). "Smoothing and Differentiation of Data by Simplified Least Squares Procedures"; Analytical Chemistry 36 (8): 1627-1639) oder gleitende Mittelwert-Glättungsfilter (The Scientist and Engineer's Guide to Digital Signal Processing, Chapter 15- Moving Average Filters, by Steven W. Smith, 11/17/2001) zur weiteren Verarbeitung optimiert. Wobei die Glättung auch mit einer Ableitung ergänzt werden kann. Im zweiten Schritt der Datenvorverarbeitung wird der gesamte Datensatz mittels Kennard-Stone Algorithmus (Kennard, R. W. and Stone, L. A. (1969) Computer aided design of experiments. Technometrics 11(1), 137-148) (ebenfalls PLS Toolbox Version 7.9.1, Eigenvector Research, Inc. Wenatchee, Vereinigte Staaten von Amerika basierend auf Matlab® 2014b, The MathWorks GmbH, Ismaning, Deutschland) in zwei unabhängige Datensätze gespalten. Die mit dem Kennard-Stone Algorithmus auserwählten Daten werden zur Erstellung des Klassifikationsmodells herangezogen, mit dem Datensatz der restlichen Daten wird validiert.

Zur Klassifizierung kann auf verschiedene Algorithmen zurückgegriffen werden, wie zum Beispiel der "k-nächste-Nachbarn-Algorithmus" (abgekürzt KNN, englisch "k-Nearest-Neighbor-Algorithmus" (Altman, N. S. (1992). "An introduction to kernel and nearest-neighbor nonparametric regression". The American Statistician 46 (3): 175-185. doi:10.1080/00031305.1992.10475879)) oder die Stützvektormaschine (abgekürzt SVM, englisch: "Support Vector Machine" (Cristianini, N., and Shawe-Taylor, J. (2000). An Introduction to Support Vector Machines and Other Kernel-based Learning Methods, First Edition (Cambridge: Cambridge University Press)).

Die Beurteilung der Klassierung wird mittels den folgenden Gütefaktoren bewertet: Richtig positiv, richtig negativ, falsch positiv und falsch negativ. Da, wie oben beschrieben, die Abtrennung der Klasse "Voll" von den restlichen Klassen im Vordergrund steht, sind die Gütefaktoren wie folgt definiert: Im Falle von richtig positiv wird Saatgut der tatsächlichen Klasse "Voll" richtigerweise der Klasse "Voll" zugeordnet; bei richtig negativ wird Saatgut einer anderen tatsächlichen Klasse richtigerweise nicht der Klasse "Voll", zugeordnet; bei falsch negativ wird Saatgut der tatsächlichen Klasse "Voll" fälschlicherweise einer anderen Klasse zugeordnet; und bei falsch positiv wird Saatgut einer anderen tatsächlichen Klasse fälschlicherweise der Klasse "Voll" zugeordnet.

Die Klassierung der Saatgut-Körner aus dem Validationsdatensatz ist in Tabelle 1 gezeigt. Die Klassierung erzielt Gütefaktoren von 100% richtig positiv, 100% richtig negativ, 0% falsch positiv und 0% falsch negativ, bezogen auf die "Volle" Klasse. Werden alle Klassen miteinbezogen, sind 89% aller Körner korrekt klassiert. Diese Ergebnisse zeigen, dass Zuckerrübensaatgut-Körner innerhalb eines Kalibiers mittels der vorliegenden Erfindung mit einer hohen Güte klassiert werden können.

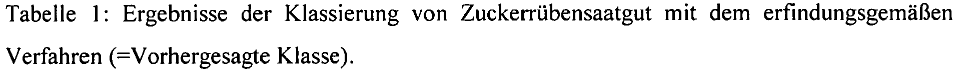

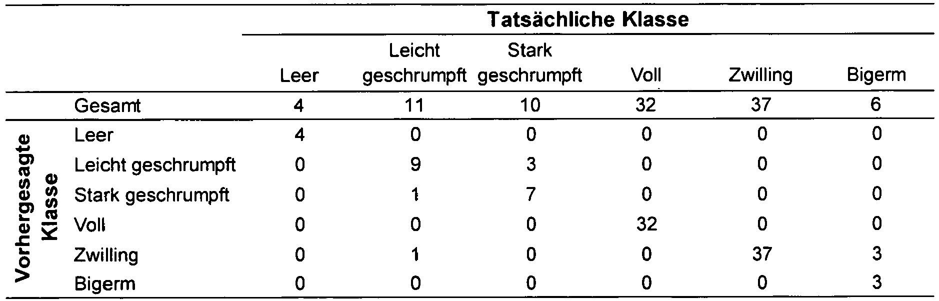

### Zwiebel und Paprika:

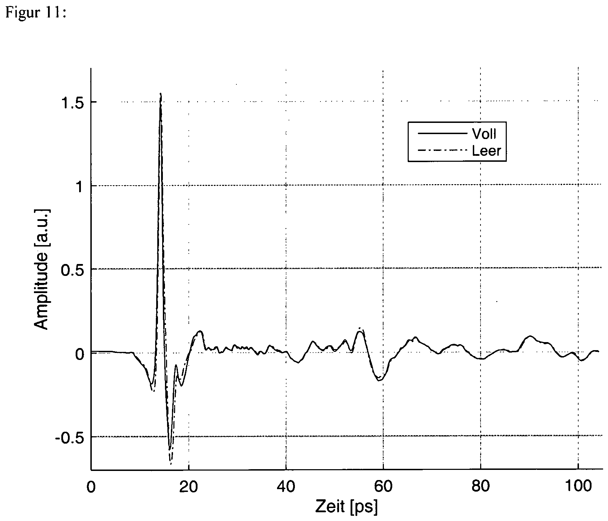

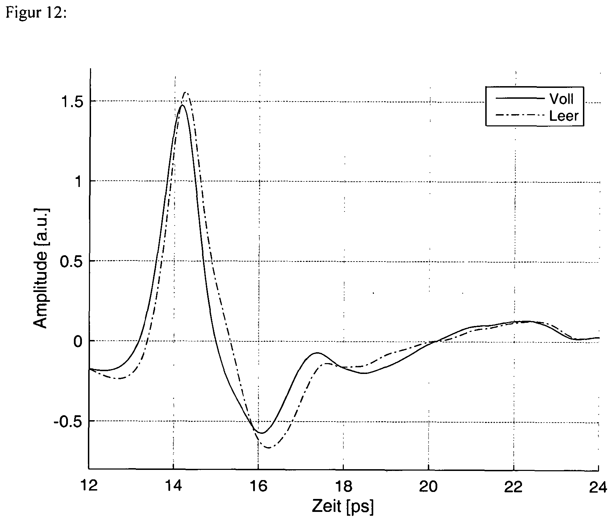

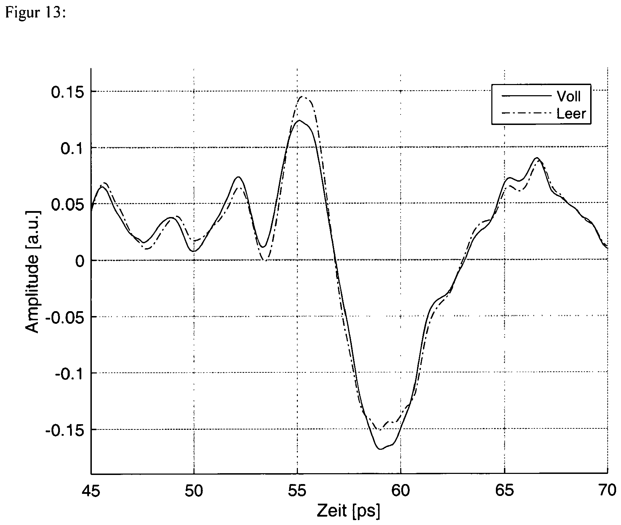

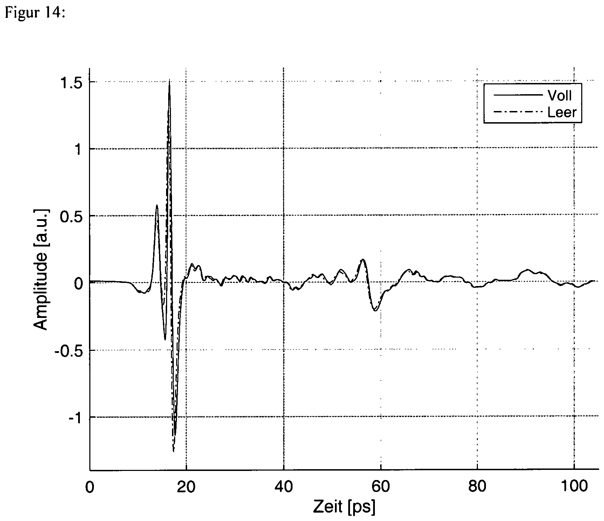

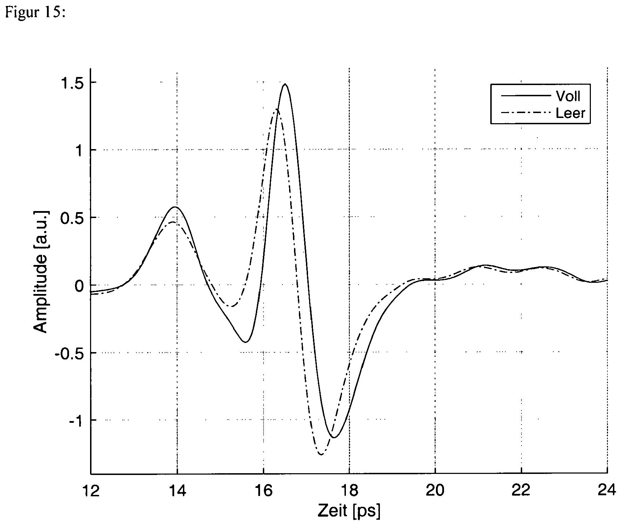

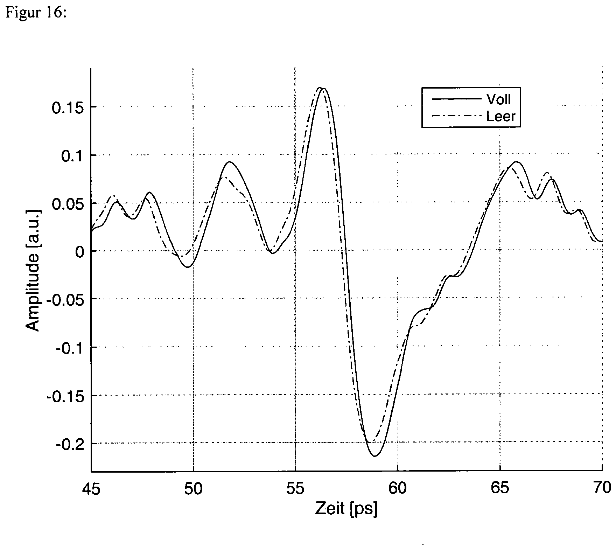

Wie oben beschrieben wird beispielhaft nur auf die Informationen der Zeitdomäne eingegangen. Die Zeitdomänenspektren des Zwiebel- und Paprikasaatgutes jeweils eines exemplarischen Kalibers sind in Figuren 11-16 dargestellt, wobei jeweils ein Mittelwertspektrum für jede Klasse gebildet wurde. Die Figuren 11 und 14 zeigen jeweils das vollständige Spektrum des Zwiebel- bzw. des Paprikasaatgutes. In den Figuren 12 und 13 für Zwiebelsaatgut sowie 15 und 16 für Paprikasaatgut sind hingegen Ausschnitte der vollständigen Spektren dargestellt, die Unterschiede in den Spektren verdeutlichen.

Die Datenvorverarbeitung und -auswertung des Zwiebel- und Paprikadatensatzes verlief analog zu dem der Zuckerrübe. Zuerst wurde eine Hauptkomponentenanalyse berechnet, anschließend, wie oben beschrieben, die Daten mit einem Glättungsfilter und eventuell mit einer Ableitung optimiert und den jeweiligen Datensatz mittels Kennard-Stone Algorithmus in zwei unabhängige aufgeteilt. Die Hauptkomponentenanalyse des Paprikasaatgutes wurde jedoch nicht mit den Rohdaten, sondern den schon optimierten Daten berechnet.

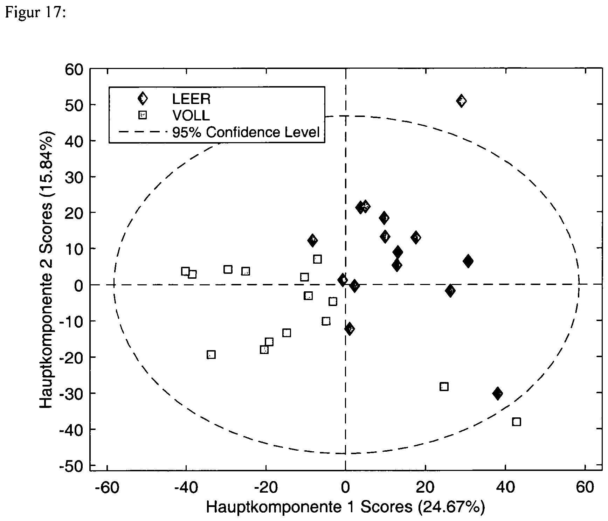

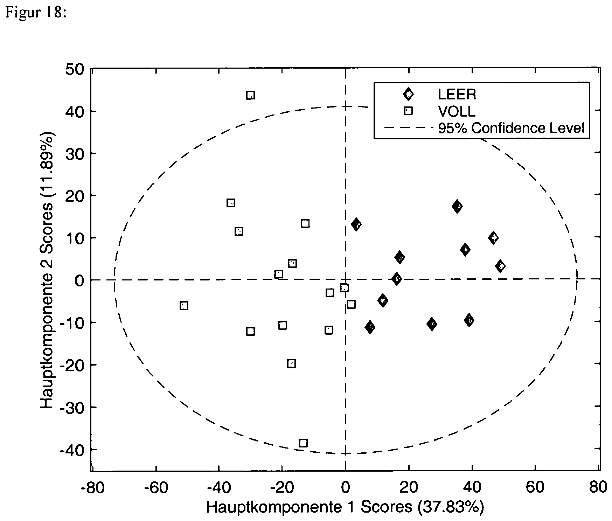

Das Score-Diagramm der Hauptkomponentenanalyse des Zwiebelsaatgutes ist in Figur 17 dargestellt. Eine Trennung der beiden Klassen "Voll" und "Geschrumpft" ist erkennbar, allerdings liegen die Datenpunkte der beiden Gruppen nah beieinander. Bei der Hauptkomponentenanalyse von Paprika, dargestellt in Figur 18, sieht die Situation ähnlich aus. Auch hier ist eine Trennung der beiden Klassen "Voll" und "Geschrumpft" erkennbar, jedoch liegen die beiden Gruppen der Datenpunkte ebenfalls nah beieinander.

Dass die Signalunterschiede bei der Zwiebel- und Paprikasaatgut kleiner ausfallen als bei der Zuckerrübe ist wahrscheinlich auf die Größe/Geometrie des Saatgutes und deren Embryonen zurückzuführen. Die Zwiebelsaatgut-Körner sind deutlich kleiner als die der Zuckerrübe, das Paprikasaatgut ist deutlich flacher.

Eine Klassifikation von Saatgut der Zwiebel und Paprika ist mittels des erfindungsgemäßen Verfahrens möglich. Die Klassierung des Validationsdatensatzes vom Saatgut der Zwiebel ist in Tabelle 2 gezeigt. Die Güte der Klassierung beträgt 87,5% richtig positiv, 12,5% falsch negativ, 14,3% falsch positiv und 85,7% richtig negativ. Insgesamt werden 86,7% der Zwiebelsaatgut-Körner korrekt klassiert. Die Klassierung des Validationsdatensatz vom Saatgut der Paprika ist in Tabelle 3 gezeigt. Alle Saatgut-Körner wurden korrekt klassiert, mit Gütefaktoren von 100% richtig positiv, 0% falsch negativ, 0% falsch positiv und 100% richtig negativ. Insgesamt werden 100% der Paprikasaatgut-Körner korrekt klassiert.

**Tabelle 2: Ergebnisse der Klassierung von Zwiebelsaatgut mit dem erfindungsgemäßen Verfahren (=Vorhergesagte Klasse).**

| | | **Tatsächliche Klasse** | |
|---|---|---|---|
| | | Geschrumpft | Voll |
| | Gesamt | 7 | 8 |
| **Vorhergesagte Klasse** | Geschrumpft | 6 | 1 |
| | Voll | 1 | 7 |

**Tabelle 3: Ergebnisse der Klassierung von Paprikasaatgut mit dem erfindungsgemäßen Verfahren (=Vorhergesagte Klasse).**

| | | **Tatsächliche Klasse** | |
|---|---|---|---|
| | | Geschrumpft | Voll |
| | Gesamt | 5 | 8 |
| **Vorhergesagte Klasse** | Geschrumpft | 5 | 0 |
| | Voll | 0 | 8 |

### Sonnenblume:

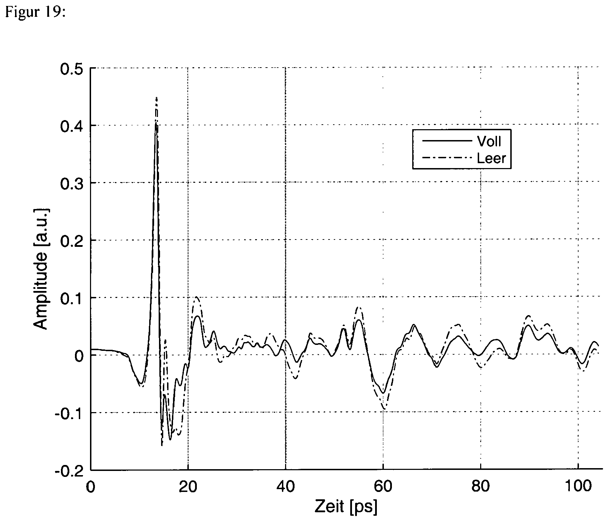

Die Zeitdomänenspektren des Sonnenblumensaatgutes sind in Figur 19 gezeigt. Da im Falle der Sonnenblume das Saatgut nicht in Kaliber unterteilt wurde, zeigt Figur 19 die Mittelwertspektren der beiden Klassen.

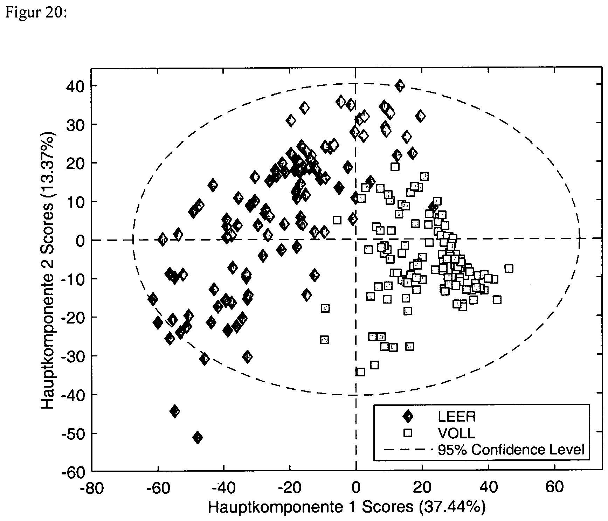

Die Datenvorverarbeitung und -auswertung erfolgt wie für Zuckerrübe, Paprika und Zwiebel ausgeführt. Die Hauptkomponentenanalyse des Sonnenblumensaatgutes wurde jedoch nicht mit den Rohdaten, sondern den schon optimierten Daten berechnet. Das Score-Diagramm der Hauptkomponente 1 und 2 sind in Figur 20 dargestellt. Auch hier findet grundsätzlich eine Trennung der beiden Klassen "Voll" und "Leer" statt, aber die aneinandergrenzenden Datenpunkte überlappen.
Die Klassierung des Validationsdatensatz vom Saatgut der Sonnenblume ist in Tabelle 4 gezeigt. Die Güte der Klassierung beträgt 98,9% richtig positiv, 1,1% falsch negativ, 0% falsch positiv und 100% richtig negativ. Insgesamt werden 99,1% der Sonnenblumensaatgut-Körner korrekt klassiert.

**Tabelle 4: Ergebnisse der Klassifikation von Sonnenblumensaatgut mit dem erfindungsgemäßen Verfahren (=Vorhergesagte Klasse).**

| | | **Tatsächliche Klasse** | |
|---|---|---|---|
| | | Geschrumpft | Voll |
| | Gesamt | 23 | 91 |
| **Vorhergesagte Klasse** | Geschrumpft | 23 | 1 |
| | Voll | 0 | 90 |

Diese Ergebnisse zeigen, dass das erfindungsgemäße Verfahren nicht nur an Zuckerrübensaatgut, sondern auch Saatgut anderer Fruchtarten, wie beispielhaft den hier gezeigten, angewendet werden kann. Zusätzlich wurde gezeigt, dass je nach Beschaffenheit der Körner auch eine Klassierung ohne vorherige Einteilung in Kaliber möglich ist.

## Patentansprüche

1. Verfahren zur Charakterisierung von morphologischen Strukturen des Zuckerrüben-Saatguts unter Verwendung der Terahertz-Zeitbereich-Spektroskopie, umfassend die Schritte:
- Beaufschlagen des Saatgut-Korns von Zuckerrübe mit einem Terahertz-Puls,
- Messen des Signals, das der Terahertz-Puls nach Transmission durch das Saatgut-Korn erzeugt,
- Bestimmen der durch die Transmission bedingten Amplitude, zeitlichen Verzögerung, Phase und/oder des Spektrums des Signals und
- Zuordnen des Saatgut-Korns zu der vorbestimmten Saatgutklasse basierend auf der Amplitude, zeitlichen Verzögerung, Phase und/oder des Spektrums des Signals und einteilen des Saatguts von Zuckerrübe in sechs verschiedene Klassen:
Klasse 1 - leere Körner (ohne Embryo)
Klasse 2 - volle Körner (mit einem vollständig ausgebildeten Embryo)
Klasse 3 - leicht geschrumpft (mit einem leicht geschrumpften Embryo)
Klasse 4 - stark geschrumpft (mit einem stark geschrumpften Embryo)
Klasse 5 - Zwilling (mit 2 Embryos in einer Samenschale)
Klasse 6 - Bigerme (mit 2 Embryos in zwei Samenschalen).

2. Verfahren nach Anspruch 1, **dadurch gekennzeichnet, dass** die morphologischen Strukturen ausgewählt sind aus dem Vorhandensein bestimmter struktureller Elemente im Saatkorninneren, vorzugsweise das Vorhandensein eines Embryos, oder die Anordnung von strukturellen Elementen im Saatkorninneren, vorzugsweise zwei Embryonen in einer oder in zwei Samenschalen.

## Claims

1. A method for characterizing morphological structures of sugar beet seeds using terahertz time-domain spectroscopy, comprising the steps:
- applying a terahertz pulse to the seed grain of sugar beet,
- measuring the signal generated by the terahertz pulse after transmission through the seed grain,
- determining the amplitude, time lag, phase and/or spectrum of the signal due to the transmission, and
- assigning the seed grain to a predetermined seed class based on the amplitude, time lag, phase and/or spectrum of the signal and dividing the seed into six different classes:
class 1 - empty grains (no embryo)
class 2 - full grains (with a fully developed embryo)
class 3 - slightly shrunken or shriveled (with a slightly shrunken embryo)
class 4 - severely shrunken or shriveled (with a severely shrunken embryo)
class 5 - twin (with 2 embryos in one seed coat)
class 6 - bigerms (with 2 embryos in two seed coats).

2. The method of claim 1, wherein the morphological structures are selected from the presence of certain structural elements in the interior of the seed grain, preferably the presence of an embryo, or the arrangement of structural elements in the interior of the seed, preferably two embryos in one seed coat or in two seed coats.

## Revendications

1. Procédé, destiné à caractériser des structures morphologiques de la semence de la betterave sucrière, en utilisant la spectroscopie dans le domaine temporel du térahertz, comprenant les étapes consistant à :
- soumettre la graine de semence de la betterave sucrière à une impulsion térahertz,
- mesurer le signal que génère l'impulsion térahertz après la transmission par la graine de semence,
- déterminer l'amplitude, le retard temporel, la phase et/ou le spectre du signal dus à la transmission, et
- associer la graine de semence de la betterave à la classe de semence prédéfinie, sur la base de l'amplitude, du retard temporel, de la phase et/ou du spectre du signal et répartir la semence de la betterave sucrière dans dix classes différentes :
Classe 1 - les graines vides (dépourvues d'embryon)
Classe 2 - les graines pleines (pourvues d'un embryon totalement constitué)
Classe 3 - légèrement rétractées (pourvues d'un embryon légèrement rétracté)
Classe 4 - fortement rétractées (pourvues d'un embryon fortement rétracté)
Classe 5 - gémellaires (pourvues de deux embryons dans un tégument)
Classe 6 - bigermes (pourvues de deux embryons dans deux téguments).

2. Procédé selon la revendication 1, **caractérisé en ce que** les structures morphologiques sont sélectionnées à partir de la présence d'éléments structurels déterminés à l'intérieur de la graine de semence, de préférence de la présence d'un embryon, ou de la disposition d'éléments structurels à l'intérieur de la graine de semence, de préférence de deux embryons dans un ou dans deux téguments.
